# EUROPEAN PATENT APPLICATION

(11) **EP 2 308 451 A1**
(43) Date of publication of application: **13.04.2011**
(21) Application number: 09804730.1
(22) Date of filing: 04.08.2009
(51) Int. Cl.: A61H 33/00, A61H 23/02

(54) **ULTRASONIC TREATMENT DEVICE**

(30) Priority: 05.08.2008 JP 2008201866
(71) Applicant: Miyake, Haru, Nagoya-shi, Aichi 465-0091 (JP)
(72) Inventor: MIYAKE, Takamura, Toyota-shi Aichi 470-0309 (JP); MIYAKE, Haru, Aichi 4650091 (JP)
(74) Representative: Bauer, Clemens
(86) International application number: PCT/JP2009/003723
(87) International publication number: WO 2010/016243

(57) **Abstract**

Bacteria, viruses, and the like can be killed by irradiating portions to be treated on the surface of the skin of a person to be treated and in the body thereof with ultrasonic waves. An ultrasonic treatment device comprises a treatment bath (11) for accommodating a medium, a first ultrasonic generation element disposed at a predetermined position in the treatment bath (11), and a vibrator unit (34) disposed so as to be movable in the treatment bath (11). The vibrator unit (34) is provided with a housing which forms a closed chamber with a predetermined portion serving as a contact portion that is brought into contact with a portion to be treated, and a second ultrasonic generation element which is attached to the contact portion in the chamber and on the back surface of which a hollow portion is formed. Not only bacteria, viruses, and the like can be killed by irradiating the portion to be treated on the surface of the skin with ultrasonic waves by driving the first ultrasonic generation element, but also bacteria, viruses, and the like can be killed by irradiating the portion to be treated in the body with ultrasonic waves by driving the second ultrasonic generation element.

## Description

### TECHNICAL FIELD

The present invention relates to an ultrasonic treatment apparatus.

### BACKGROUND ART

Conventionally, there has been provided an ultrasonic treatment apparatus for treating a whole body of a person to be treated with ultrasonic waves. The ultrasonic treatment apparatus includes a bathtub and an ultrasonic vibrator disposed in the bathtub, and ultrasonic waves generated by the ultrasonic vibrator are radiated onto the whole body (see, for example, Patent Document 1).
Patent document 1: Japanese Patent Application Laid-Open (Kokai) No. H07-469

### DISCLOSURE OF THE INVENTION

### PROBLEMS TO BE SOLVED BY THE INVENTION

However, since conventional ultrasonic treatment apparatuses, primarily designed for cosmetic purposes, radiate ultrasonic waves onto only a skin surface, they cannot irradiate a part to be treated inside the body, such as internal organs, to thereby sterilize bacteria, viruses and the like.

In view of the foregoing, an object of the present invention is to solve the above-mentioned problem of conventional ultrasonic treatment apparatuses, and to provide an ultrasonic treatment apparatus with which ultrasonic waves can be radiated onto a part to be treated on a skin surface or inside the body of a person to be treated to thereby sterilize bacteria, viruses and the like.

### MEANS FOR SOLVING THE PROBLEMS

In order to achieve the aforementioned object, the ultrasonic treatment apparatus of the present invention comprises a treatment vessel for accommodating a medium; a first ultrasonic generating element disposed at a predetermined location in the treatment vessel; and a vibrator unit movably disposed in the treatment vessel.

The vibrator unit comprises a housing forming a sealed chamber and having a predetermined location thereof serving as an abutment section to be abutted against a part to be treated, and a second ultrasonic generating element attached to the abutment section in the chamber and having a hollow section formed on a back face thereof.

### EFFECTS OF THE INVENTION

According to the present invention, the ultrasonic treatment apparatus comprises a treatment vessel for accommodating a medium; a first ultrasonic generating element disposed at a predetermined location in the treatment vessel; and a vibrator unit movably disposed in the treatment vessel.

The vibrator unit comprises a housing forming a sealed chamber and having a predetermined location thereof serving as an abutment section to be abutted against a part to be treated, and a second ultrasonic generating element attached to the abutment section in the chamber and having a hollow section formed on a back face thereof.

In this case, since the first ultrasonic generating element is disposed at a predetermined location in the treatment vessel and the vibrator unit is movably disposed in the vessel, not only a part to be treated on a skin surface can be irradiated with ultrasonic waves by driving the first ultrasonic generating element to thereby sterilize bacteria, viruses and the like, but also a part to be treated inside the body can be irradiated with ultrasonic waves by driving the second ultrasonic generating element to thereby sterilize bacteria, viruses and the like.

Since the air in the hollow section on the back face of the second ultrasonic generating element prevents transmission of ultrasonic waves, the waves are not transmitted backward. Therefore, the ultrasonic waves can be effectively radiated onto a part to be treated.

### BRIEF DESCRIPTION OF THE DRAWINGS

[Fig. 1] Schematic diagram of an ultrasonic treatment apparatus according to a first embodiment of the present invention.
[Fig. 2] Conceptual diagram of the ultrasonic treatment apparatus according to the first embodiment of the present invention.
[Fig. 3] Diagram showing a vibrator unit according to the first embodiment of the present invention.
[Fig. 4] Diagram showing operation of the vibrator unit according to the first embodiment of the present invention.
[Fig. 5] Control block diagram of the ultrasonic treatment apparatus according to the first embodiment of the present invention.
[Fig. 6] Diagram showing how the vibrator unit is disposed according to a second embodiment of the present invention.
[Fig. 7] Schematic diagram of the ultrasonic treatment apparatus according to a third embodiment of the present invention.
[Fig. 8] Schematic diagram of another ultrasonic treatment apparatus according to the third embodiment of the present invention.
[Fig. 9] Schematic diagram of the ultrasonic treatment apparatus according to a fourth embodiment of the present invention.
[Fig. 10] Control block diagram showing a relevant part of the ultrasonic treatment apparatus according to a fifth embodiment of the present invention.

### DESCRIPTION OF REFERENCE NUMERALS

10, 90: ultrasonic treatment apparatus
11, 91: treatment vessel
31, 39: ultrasonic vibrator
34: vibrator unit
35, am1, am2, am3: arm
45: bottomed tubular member
46: closure member
51: chamber

### BEST MODE FOR CARRYING OUT THE INVENTION

Embodiments of the present invention will next be described in detail with reference to the drawings.

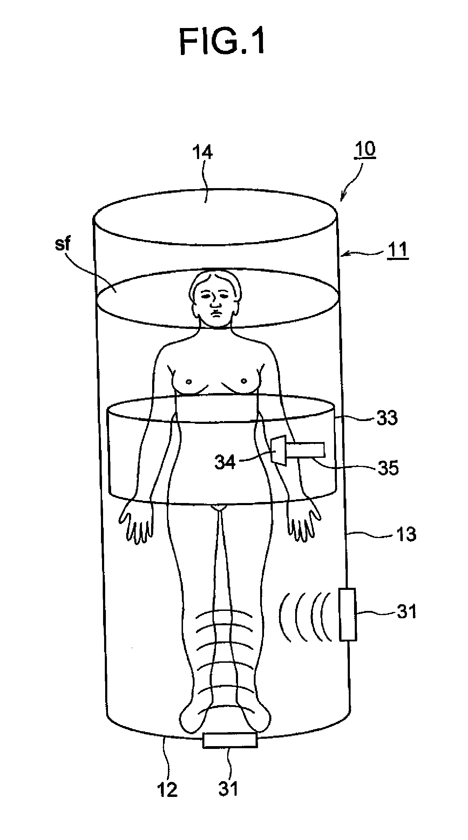

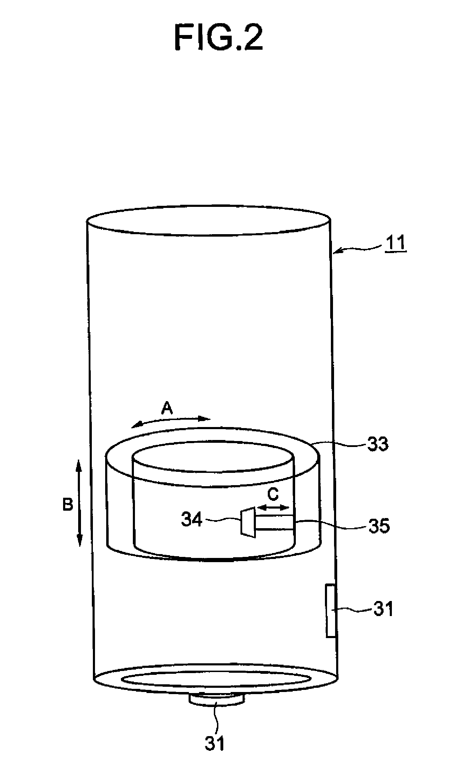

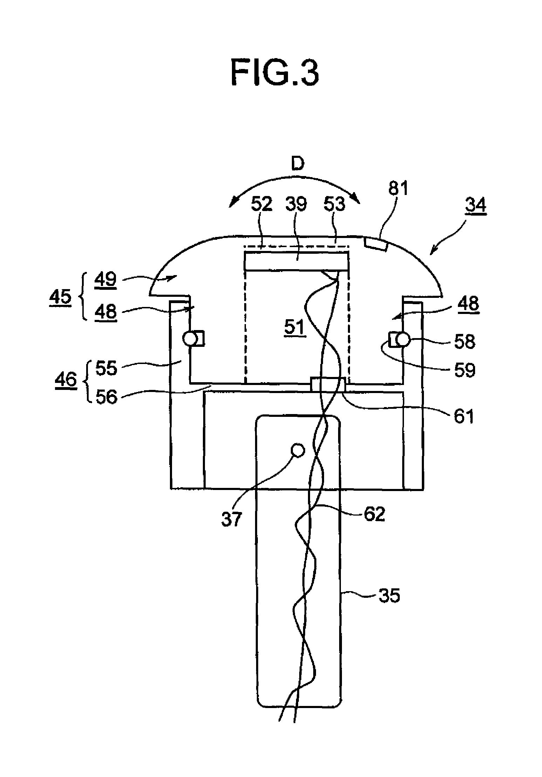

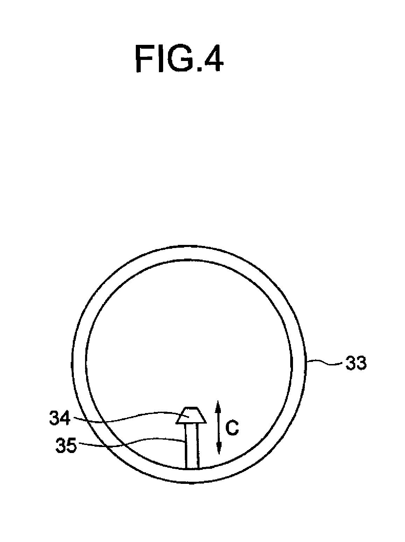

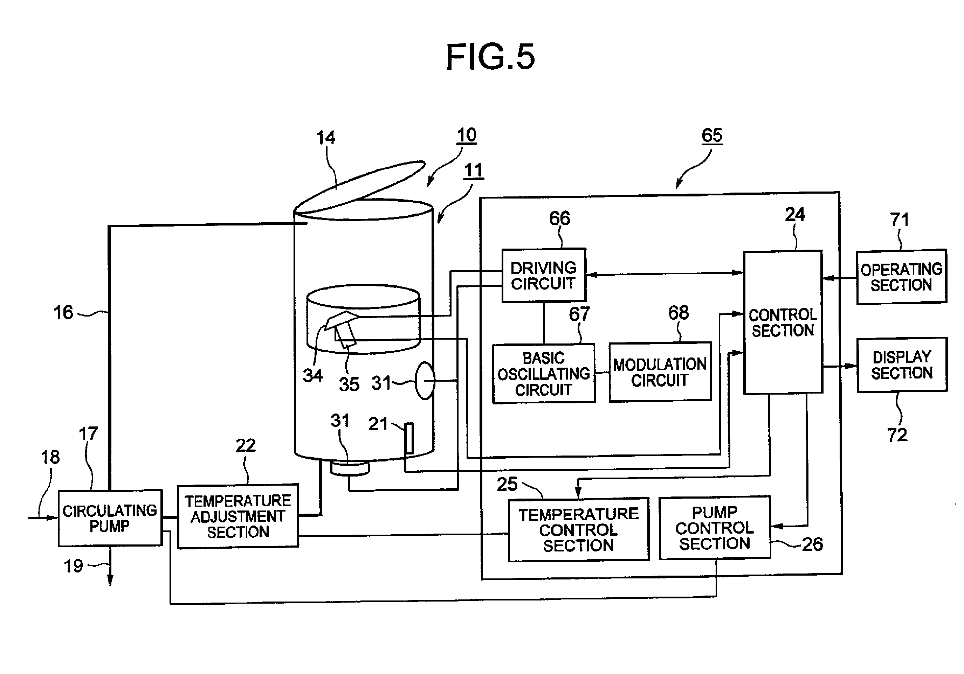

Fig. 1 is a schematic diagram of an ultrasonic treatment apparatus according to a first embodiment of the present invention. Fig. 2 is a conceptual diagram of the ultrasonic treatment apparatus according to the first embodiment of the present invention. Fig. 3 is a diagram showing a vibrator unit according to the first embodiment of the present invention. Fig. 4 is a diagram showing operation of the vibrator unit according to the first embodiment of the present invention. Fig. 5 is a control block diagram of the ultrasonic treatment apparatus according to the first embodiment of the present invention.

In these figures, reference numeral 10 denotes a capsular ultrasonic treatment apparatus, and reference numeral 11 denotes a treatment vessel formed in a predetermined shape (in the present embodiment, cylindrical shape) including a bottom wall 12, a tubular side wall 13, and a top wall 14 to constitute a sealed capsule. The treatment vessel 11 is made of transparent materials, such as acrylic resin and glass, and is fabricated by press molding, injection molding or the like.

The top wall 14 is openably/closably provided, so that a person to be treated by ultrasonic waves can enter or leave the treatment vessel 11 by opening the top wall 14. Notably, the treatment vessel 11 is provided with an unillustrated ladder therein.

The treatment vessel 11 accommodates water at a predetermined temperature as a medium, and a person to be treated stands upright within the treatment vessel 11 while soaking a diseased part (i.e., a part to be treated) in the water, e.g., keeping the head above a water surface sf. If a part to be treated is the head, a person to be treated wears an oxygen mask and soaks the whole body in the water. Notably, in order to prevent the body of a person to be treated from floating in the water, a locking section for tangling the feet is formed on the bottom wall 12, for example, to allow a person to be treated locking the feet with the locking section.

In order to adjust the temperature of the water in the treatment vessel 11, a temperature sensor 21 serving as a temperature detection section is disposed at a predetermined location in the treatment vessel 11 and a medium circulation passage 16 is connected to the treatment vessel 11, the medium circulation passage 16 being provided with a circulating pump 17 and a temperature adjustment section 22. The circulating pump 17 circulates the water discharged from the treatment vessel 11 through the medium circulation passage 16, and the temperature adjustment section 22 adjusts the temperature of the water to a predetermined temperature until it is returned to the treatment vessel 11. For this purpose, the temperature adjustment section 22 includes unillustrated heater and cooler to heat or cool the water to the predetermined temperature. The circulating pump 17 is connected with a medium supply passage 18 for supplying new water to the treatment vessel 11 from the outside and a medium discharge passage 19 for discharging the water from the treatment vessel 11 to the outside.

The present embodiment is constituted to generate hydroxyl radicals by ultrasonic waves, treat the whole body of a person to be treated, and radiate the ultrasonic waves not only onto a part to be treated outside the body, e.g., a skin surface, but also a part to be treated inside the body, such as a subcutaneous region near the skin surface and internal organs, to allow sterilization of bacteria, viruses and the like.

For this purpose, an ultrasonic vibrator 31 serving as a first ultrasonic generating element is disposed on a predetermined wall (in the present embodiment, the bottom wall 12 and the side wall 13) of the treatment vessel 11. At least one ultrasonic vibrator 31 is provided while being fixed to the treatment vessel 11 to constitute a first ultrasonic generating section for generating ultrasonic waves, and the generated ultrasonic waves are transmitted through the water toward a preliminarily set direction.

A vibrator unit 34 is also movably disposed in the treatment vessel 11 and pressed against a predetermined site of the skin, i.e., a pressed region. For this purpose, an annular body 33 serving as a moving support is provided rotatably in the circumferential direction (direction of arrow A) of the treatment vessel 11 and vertically movably in the axial direction (direction of arrow B) of the treatment vessel 11. On the inner circumferential surface of the annular body 33, at least one vibrator unit 34 is provided movably forward/backward in the radial direction (direction of arrow C) of the treatment vessel 11 via an arm 35 serving as a support member. The arm 35 is attached movably forward/backward to the inner circumferential surface of the annular body 33. The vibrator unit 34 is supported swingably with respect to the arm 35 in the direction of arrow D about a shaft 37. The vibrator unit 34 and the arm 35 constitute a second ultrasonic generating section.

The inner diameter of the annular body 33 is set to allow a predetermined movement of a person to be treated within the annular body 33. Although two ultrasonic vibrators 31 and one vibrator unit 34 are provided in the present embodiment, any number of them can be provided respectively. An unillustrated rotating mechanism for rotating the annular body 33, an unillustrated elevating mechanism for moving the annular body 33 vertically, and an unillustrated moving mechanism for advancing and retracting the vibrator unit 34 are each disposed at a predetermined location in the treatment vessel 11. By providing motors serving as driving sections to the rotating mechanism, elevating mechanism, and moving mechanism, and driving the motors, the rotating mechanism, elevating mechanism, and moving mechanism can be automatically actuated. The rotating mechanism, elevating mechanism, and moving mechanism may also be actuated manually. Notably, the rotating mechanism, elevating mechanism, and moving mechanism constitute first to third actuating mechanisms, respectively.

The vibrator unit 34 comprises a bottomed tubular member 45 serving as a first housing, a closure member 46 serving as a second housing, and an ultrasonic vibrator 39 disposed in the bottomed tubular member 45 and serving as a second ultrasonic generating element. The bottomed tubular member 45 comprises a tubular section 48 and a closure section 49 for closing the front end of the tubular section 48, and the closure section 49 constitutes an abutment section to be abutted against the pressed region at a predetermined location of the vibrator unit 34 (in the present embodiment, the front end of the vibrator unit 34).

In the present embodiment, the vibrator unit 34 is made of a rust-resistant material having no effect on the human body, such as stainless steel. Although each of the ultrasonic vibrators 31 and 39 is made of stainless steel in the present embodiment, they may be made of a material which transmits vibrations, such as glass and aluminum.

In the bottomed tubular member 45, a columnar chamber 51 is formed by the tubular section 48 and the closure section 49, and the ultrasonic vibrator 39 is attached to the back face of the closure section 49 in the chamber 51 by an adhesive 52 serving as a fixing member.

In the closure section 49, a thin-walled portion to which the ultrasonic vibrator 39 is attached forms a resonant section 53. The thickness of the resonant section 53 is set so as to resonate along with driving of the ultrasonic vibrator 39.

The closure member 46 comprises a closure section 56 which closes a tubular section 55 surrounding the tubular section 48 and an intermediate section with the tubular section 55, and which supports the rear end of the tubular section 48 and seals the chamber 51. Between the tubular sections 48 and 55, an O-ring 58 serving as a first sealing member is accommodated in a groove 59 formed on the outer circumferential surface of the tubular section 48 to seal the chamber 51.

On the closure section 56, a cord bushing 61 facing the chamber 51 and serving as a second sealing member is disposed, and a cord 62 for supplying power to the ultrasonic vibrator 39 is wired through the cord bushing 61.

Next, a control device 65 of the ultrasonic treatment apparatus 10 will be described.

As shown in Fig. 5, the control device 65 comprises a control section 24 for controlling the ultrasonic treatment apparatus 10 as a whole, a pump control section 26 for controlling the circulating pump 17, a temperature control section 25 for controlling the temperature adjustment section 22, a driving circuit 66 for driving each of the ultrasonic vibrators 31 and 39, a basic oscillating circuit 67, a modulation circuit 68, and the like. The control section 24 is connected with an operating section 71 and a display section 72.

The control section 24 comprises an unillustrated CPU serving as a computing unit, an unillustrated RAM used as a working memory when the CPU performs various types of operational processing, a control program, an unillustrated ROM on which various types of data is recorded, and the like. The CPU functions as a computer based on various types of programs, data and the like. The RAM, ROM and the like constitute a recording unit. The CPU can be replaced with an MPU or the like as a computing unit.

The control section 24 reads the temperature of the water detected by the temperature sensor 21, sends an instruction to the temperature control section 25 to adjust the temperature of the water by actuating the temperature adjustment section 22, and sends an instruction to the pump control section 26 to circulate the water by actuating the circulating pump 17.

Moreover, the control section 24 sends an instruction to the pump control section 26 if the ultrasonic treatment apparatus 10 malfunctions, so as to actuate the circulating pump 17 to discharge the water from the treatment vessel 11 while releasing the locking by the locking section. Notably, the water can be discharged from the treatment vessel 11 in a short time by providing an unillustrated openable/closable drain serving as an emergency drain on the bottom wall 12 and opening the drain when a malfunction occurs.

The driving circuit 66 is connected with each of the ultrasonic vibrators 31 and 39 via an unillustrated trans, and an unillustrated driving processing means (driving processing section) of the control section 24 performs driving processing, sends a control signal to the driving circuit 66, and drives each of the ultrasonic vibrators 31 and 39 at a predetermined frequency (in the present embodiment, a controlled frequency not lower than 950 kHz but not greater than 2 MHz) to generate ultrasonic waves within a range of 950 kHz to 2 MHz. In the present embodiment, the output of the ultrasonic waves is set so that the amount of generated hydroxyl radicals falls within a range of 0.1 µM to 60 µM.

The trans is provided for adjusting impedance, and for insulating between each of the ultrasonic vibrators 31 and 39 and the control section 24 or the like.

The basic oscillating circuit 67 performs basic oscillation based on a signal having a frequency of 1. 65 MHz generated by the modulation circuit 68.

When ultrasonic waves generated by each of the ultrasonic vibrators 31 and 39 are transmitted through the water, the water is decomposed by the ultrasonic waves to thereby generate hydroxyl radicals and hydrogen atoms. In addition, when the ultrasonic waves are transmitted to a human body indirectly via the water or directly, the water in the body is similarly decomposed by the ultrasonic waves to thereby generate hydroxyl radicals and hydrogen atoms. Therefore, bacteria, viruses and the like can be sterilized by irradiating a part to be treated with the ultrasonic waves.

In the present embodiment, for effective treatment of a part to be treated, the amount of generated hydroxyl radicals falls within a range of 0.1 µM to 80 µM, which is a value measured by an electron spin resonance (ESR) spin trapping method (apparatus) with 5,5-dimethyl-1-pyrroline-N-oxide (DMPO).

The driving time of each of the ultrasonic vibrators 31 and 39 is one second or more, and is set to a time that does not affect cells and tissues in the human body. The higher the output of ultrasonic waves, the shorter the driving time.

Since diseases of the whole body exhibit a variety of symptoms, in the present embodiment, an irradiation method of ultrasonic waves is changed depending on a location of a part to be treated to thereby perform an effective treatment.

That is, when treating the skin surface, a subcutaneous region near the skin surface, or the like, ultrasonic waves are radiated through the water. For this purpose, it is preferred to drive the ultrasonic vibrator 31 at a position away from the human body (approximately 10 mm away from the skin surface) and generate ultrasonic waves to irradiate a part to be treated.

For example, in the treatment of a subcutaneous region near the skin surface, bacteria, viruses and the like in the blood can be sterilized by irradiating intravascular (intravenous) blood with ultrasonic waves. Although a dialysis treatment requires the blood to outflow from the body to remove bacteria, viruses and the like using a filter or the like, the present embodiment allows sterilization of bacteria, viruses and the like in the blood without the need for the blood to outflow from the body. In addition, bacteria, viruses and the like inside the body can be sterilized via the blood. Notably, for the ultrasonic vibrator 31, the generated ultrasonic waves can be reflected and spread by an unillustrated reflecting plate.

Meanwhile, when treating internal organs located further inside than a subcutaneous region (for example, treating liver, e.g., hepatitis C) , in an attempt to irradiate a part to be treated inside the body with ultrasonic waves generated by the ultrasonic vibrator 31, the water exists between the ultrasonic vibrator 31 and a person to be treated, and thus the ultrasonic waves are radiated onto a part to be treated inside the body through the water. In this case, since the ultrasonic waves are reflected on the skin surface, they cannot sufficiently irradiate a part to be treated inside the body.

In order to solve the problem, in the present embodiment, the vibrator unit 34 is used when irradiating a part to be treated inside the body with ultrasonic waves to press an end of the vibrator unit 34 (closure section 49) against the skin with a constant pressure so that the ultrasonic waves are not reflected on the skin surface. For this purpose, a pressure sensor 81 serving as a pressure detection section is disposed at a predetermined location of the bottomed tubular member 45 (in the present embodiment, in the vicinity of the resonant section 53 of the closure section 49). The pressure detected by the pressure sensor 81 is sent to the control section 24, then an unillustrated pressure adjustment processing means (pressure adjustment processing section) of the control section 24 performs pressure adjustment processing and drives the motor of the moving mechanism so as to match the detected pressure to a preliminarily set pressure, i.e., a set pressure.

Thereby, since ultrasonic waves are not reflected on the skin surface, a part to be treated inside the body can be irradiated sufficiently with the ultrasonic waves.

Notably, when the ultrasonic vibrator 39 is driven, ultrasonic waves generated at the front face of the ultrasonic vibrator 39 resonate the resonant section 53 and are then transmitted forward through the water. On the other hand, ultrasonic waves generated at the rear face of the ultrasonic vibrator 39 are prevented from transmitting due to the presence of the air in the hollow section formed by the chamber 51, so that the ultrasonic waves are not transmitted backward. Therefore, a part to be treated can be irradiated effectively with the ultrasonic waves. Although the chamber 51 is filled with the air in the present embodiment, the chamber 51 may be vacuumed to prevent ultrasonic waves from transmitting therethrough.

As described above, in the present embodiment, since the ultrasonic vibrator 31 is disposed at a predetermined location in the treatment vessel 11 and the vibrator unit 34 is movably disposed in the vessel 11, not only a part to be treated outside the body (the skin surface) but also a part to be treated inside the body can be irradiated with ultrasonic waves to thereby sterilize bacteria, viruses and the like.

In addition, since the blood can be irradiated with the ultrasonic waves to thereby sterilize bacteria, viruses and the like in the blood without the need for the blood to outflow, the treatment of a part to be treated can be performed without causing exhaustion of a person to be treated.

Notably, although an attempt to accurately identify a part to be treated inside the body requires an apparatus for identifying a diseased part of the body, e.g., ultrasonic echographs, resulting in an increase in size of treatment equipment, in the present embodiment, the treatment vessel 11 is made of a transparent material as described above so that an operator can visually check and identify a part to be treated and can generate hydroxyl radicals and perform the treatment by irradiating the entire region around a part to be treated inside the body with ultrasonic waves. This eliminates the need for the apparatus for identifying a diseased part of the body, e.g., ultrasonic echographs, resulting in an decrease in size of the treatment equipment.

Notably, for a site which should not be irradiated with ultrasonic waves, a protector that is composed of a material which absorbs ultrasonic waves (e.g., rubber, urethane) or a material which reflects ultrasonic waves (e.g., metal) is applied for example.

Although, in the present embodiment, an end of the vibrator unit 34 is pressed against the skin at the set pressure to irradiate a part to be treated inside the body with ultrasonic waves generated by the ultrasonic vibrator 39, the ultrasonic waves may be absorbed by blood, fat, muscles, bones and the like inside the body before they reach a part to be treated depending on a location of a part to be treated inside the body. In this case, the absorption coefficient of the ultrasonic waves is approximately 0.12 dB/cm² for blood, approximately 0. 63 dB/cm² for fat, between 1.3 and 3.3 dB/cm² for muscles, and approximately 13dB/cm² for bones. The higher the absorption coefficient of the site is, the more the ultrasonic waves are absorbed.

Accordingly, the irradiation energy for a part to be treated is lessened as much as a fraction of the ultrasonic waves absorbed until they reach a part to be treated.

Thus, in the present embodiment, when each site of a part to be treated inside the body is displayed on the display section 72 and an operator operates the operating section 71 to specify (identify) a predetermined part to be treated, an unillustrated output alteration processing means (output alteration processing section) of the control section 24 performs output alteration processing, reads a current ultrasonic output from the driving circuit 66 while referring to the ROM to read out an ultrasonic output corresponding to the specified part to be treated as a target output, sends an instruction to the driving circuit 66 to match the current ultrasonic output to the target output, and drives the ultrasonic vibrator 39. The ROM records each site of a part to be treated inside the body and the corresponding target output.

Therefore, since the output can be increased by the amount corresponding to the absorbed ultrasonic waves, a part to be treated, regardless of its site, can be irradiated sufficiently with ultrasonic waves.

In the present embodiment, the annular body 33 is used for movably disposing the vibrator unit 34 in the treatment vessel 11 and the vibrator unit 34 is provided movably forward/backward with respect to the annular body 33 via a single arm 35. A second embodiment of the present invention will next be described in which, in contrast, the vibrator unit 34 is provided movably with respect to the annular body 33 via a plurality of arms. Notably, elements having the same structure as those of the first embodiment are given the same reference numerals as those thereof, and the effect of the invention achieved by having the same structure applies that of the first embodiment.

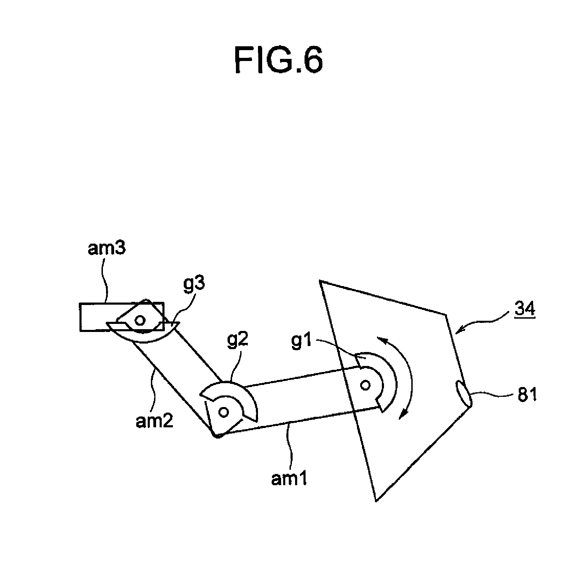

Fig. 6 is a diagram showing how the vibrator unit is disposed according to the second embodiment of the present invention.

In Fig. 6, ami (i = 1, 2, ...) denotes arms serving as support members for providing the vibrator unit 34 movably with respect to the treatment vessel 11. Gears g1, g2, and g3 are disposed respectively between the arm am1 and the vibrator unit 34, between the adjacent arms am1 and am2, and between the adjacent arms am2 and am3, wherein the gears g1, g2, and g3 are rotated by driving a motor for the moving mechanism constituting the third actuating mechanism, resulting in rocking of the vibrator unit 34 and each of the arms am1 to am3 at a predetermined angle.

Next, a third embodiment of the present invention will be described in which the vibrator unit 34 is attached to the treatment vessel 11. Notably, elements having the same structure as those of the first and second embodiments are given the same reference numerals as those thereof, and the effect of the invention achieved by having the same structure applies that of the first and second embodiments.

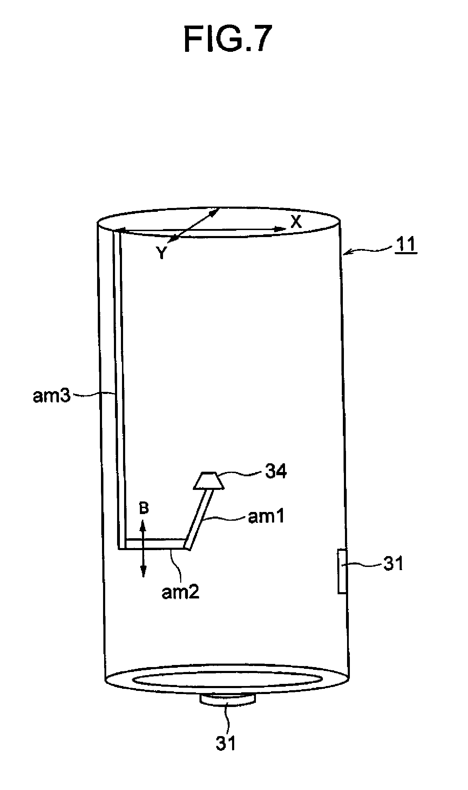

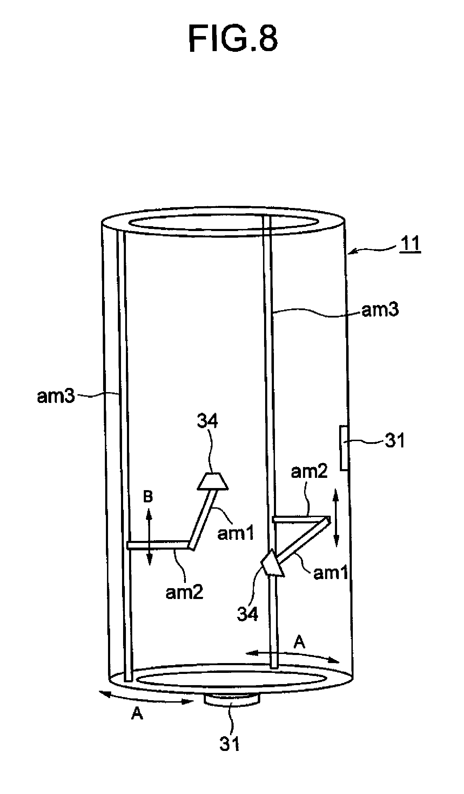

Fig. 7 is a schematic diagram of the ultrasonic treatment apparatus according to the third embodiment of the present invention. Fig. 8 is a schematic diagram of another ultrasonic treatment apparatus according to the third embodiment of the present invention.

In Fig. 7, since the arm am3 serving as a support member is provided movably in the X and Y directions as seen in the horizontal direction while the arm am2 serving as a support member is provided vertically movably in the axial direction (direction of arrow B) of the treatment vessel 11, the vibrator unit 34 can be moved to an arbitrary position in the treatment vessel 11.

In Fig. 8, since the arm am3 is provided movably in the circumferential direction (direction of arrow A) of the treatment vessel 11 while the arm am2 is provided vertically movably in the axial direction (direction of arrow B) of the treatment vessel 11, the vibrator unit 34 can be moved to an arbitrary position in the treatment vessel 11.

Next, a fourth embodiment of the present invention will be described. Notably, elements having the same structure as those of the first to third embodiments are given the same reference numerals as those thereof, and the effect of the invention achieved by having the same structure applies that of the first to third embodiments.

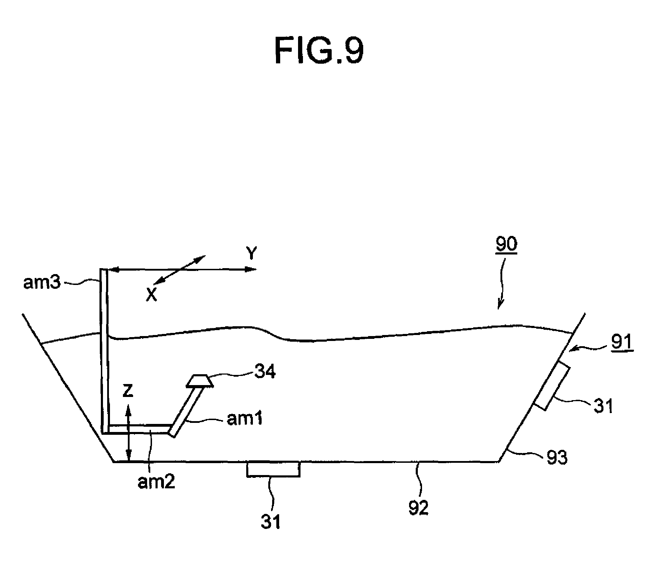

Fig. 9 is a schematic diagram of the ultrasonic treatment apparatus according to the fourth embodiment of the present invention.

In Fig. 9, reference numeral 90 denotes a bathtub-shaped ultrasonic treatment apparatus, and reference numeral 91 denotes a treatment vessel having a predetermined shape (in the present embodiment, a bathtub shape), the treatment vessel 91 having a bottom wall 92 and a side wall 93 to constitute an open-ended bathtub.

On a predetermined wall (in the present embodiment, the bottom wall 92 and the side wall 93) of the treatment vessel 91, the ultrasonic vibrator 31 serving as a first ultrasonic generating element is disposed. At least one ultrasonic vibrator 31 is provided while being fixed to the treatment vessel 91 to constitute a first ultrasonic generating section for generating ultrasonic waves, and the generated ultrasonic waves are transmitted through the water toward a preliminarily set direction.

In the treatment vessel 91, the vibrator unit 34 is also movably provided while being immersed in the water. For this purpose, the vibrator unit 34 is attached to a predetermine location in the treatment vessel 91 via the arms ami (i = 1, 2, ...) serving as a support member.

Since the arm am3 is provided movably in the X and Y directions as seen from the horizontal direction while the arm am2 is provided vertically movably in the Z direction, the vibrator unit 34 can be moved to an arbitrary position in the treatment vessel 91.

Although, in each of the above-described embodiments, a part to be treated of the human body is irradiated with ultrasonic waves to thereby sterilize bacteria, viruses and the like, the ultrasonic waves may be radiated onto a part to be treated of a living body other than the human being to thereby sterilize bacteria, viruses and the like. In that case, the treatment vessels 11 and 91 or the like are formed in a size that is sufficient to accommodate the living body.

In each of the above-described embodiments, the vibrator unit 34 is pressed against the skin to irradiate a part to be treated with ultrasonic waves generated by the ultrasonic vibrator 39, in which event a natural frequency of the ultrasonic vibrator 39 differs from that of a site of the skin against which the ultrasonic vibrator 39 is pressed, i.e., a pressed region, resulting in a change in the frequency of the ultrasonic waves generated by the ultrasonic vibrator 39.

In this case, the harder the pressed region is, the higher the natural frequency of the pressed region is, and vice versa, so that when the vibrator unit 34 is pressed against the skin, the frequency increases if the pressed region is a site closer to a bone or the frequency decreases if the region is a rather fatty site.

In this case, while the basic oscillating circuit 67 performs basic oscillation based on a signal having a frequency of 1.65 MHz generated by the modulation circuit 68, as described above, it follows a change in the frequency as the vibrator unit 34 is pressed against the skin within a range of approximately +/- 0.1 MHz to correct the frequency.

However, if the frequency changes more than the range of approximately +/- 0.1 MHz, the basic oscillating circuit 67 cannot correct the frequency, resulting in a decrease in the output of ultrasonic waves. As a result, the irradiation energy of the ultrasonic waves radiated onto a part to be treated is reduced.

A fifth embodiment of the present invention will next be described which can prevent the irradiation energy of ultrasonic waves radiated onto a part to be treated from being reduced. Notably, elements having the same structure as those of the first to fourth embodiments are given the same reference numerals as those thereof, and the effect of the invention achieved by having the same structure applies that of the first to fourth embodiments.

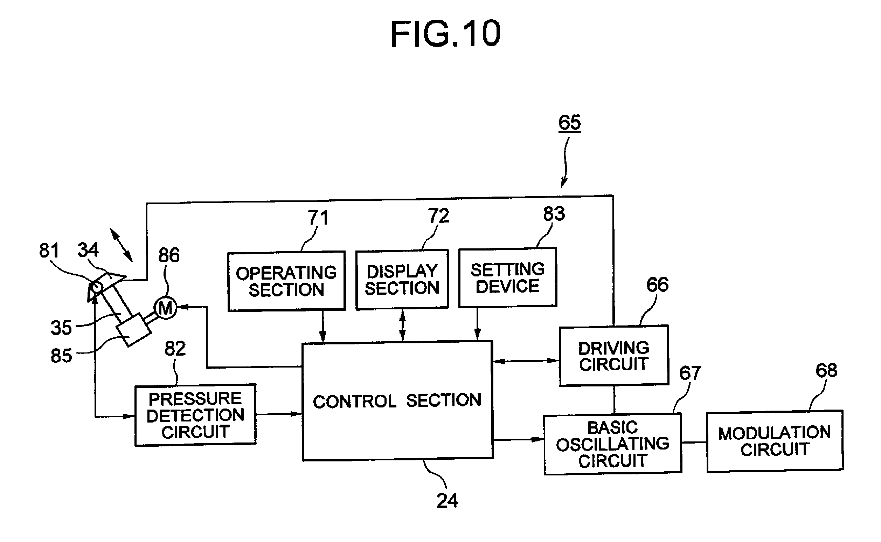

Fig. 10 is a control block diagram showing a relevant part of the ultrasonic treatment apparatus according to the fifth embodiment of the present invention.

In Fig. 10, reference numeral 24 denotes a control section, 34 denotes a vibrator unit, 35 denotes an arm serving as a support member, 65 denotes a control device, 66 denotes a driving circuit, 67 denotes a basic oscillating circuit, 68 denotes a modulation circuit, 71 denotes an operating section, 72 denotes a display section, 81 denotes a pressure sensor serving as a pressure detection section for detecting a pressure when the vibrator unit 34 is pressed against the skin, 82 denotes a pressure detection circuit for sending an instruction to detect the pressure to the pressure sensor 81, 83 denotes a setting device, 85 denotes a moving mechanism constituting the third actuating mechanism for advancing/retracting the vibrator unit 34 in the arrow direction, and 86 denotes a motor serving as a driving section for pressure adjustment.

In this case, when the vibrator unit 34 is pressed against the skin, a natural frequency of the ultrasonic vibrator 39 (Fig. 3) serving as a second ultrasonic generating element differs from that of the pressed region, and as the natural frequency of ultrasonic waves changes beyond a followable range, the output of the ultrasonic waves also changes.

Then, an unillustrated output detection processing means (output detection processing section) of the control section 24 performs output detection processing to detect a current ultrasonic output by reading it from the driving circuit 66. Subsequently, an unillustrated frequency detection processing means (frequency detection processing section) of the control section 24 performs frequency detection processing, refers to the ROM, and detects the ultrasonic frequency based on the output detected by the output detection processing means. The ROM records the ultrasonic outputs and the corresponding frequencies.

When the frequency is detected, an unillustrated frequency correction processing means (frequency correction processing section) of the control section 24 performs frequency correction processing, calculates a correction value of the frequency so that the detected ultrasonic frequency matches to a target frequency, sends the correction value to the basic oscillating circuit 67, and actuates the basic oscillating circuit 67. In this manner, the frequency can be corrected.

As described above, since the basic oscillating circuit 67 is actuated so as to match the detected frequency to the target frequency, ultrasonic waves having the target frequency can be generated. As a result, it can prevent the output of the ultrasonic waves from being reduced, and can prevent the irradiation energy of the ultrasonic waves to be radiated onto a part to be treated from being reduced.

If noises occur in reading a current ultrasonic output from the driving circuit 66, the ultrasonic output cannot be precisely detected in output detection processing, resulting in a failure in precisely detecting the frequency.

In order to solve the problem, the pressure adjustment processing means presses the vibrator unit 34 against the pressed region at the set pressure corresponding to a part to be treated. For this purpose, the pressed region is classified depending on whether it is a site closer to a bone or a rather fatty site, for example, and the pressure is set corresponding to each site and recorded on the ROM. When an operator operates the operating section 71 to input a site of the pressed region, the pressure adjustment processing means refers to the ROM and reads out the pressure corresponding to the input site as the set pressure. Subsequently, the pressure adjustment processing means drives the motor 86, actuates the moving mechanism 85, and advances/retracts the vibrator unit 34 via the arm 35. The pressure detected by the pressure sensor 81 is sent to the control section 24 as the detected pressure, and the pressure adjustment processing means drives the motor 86 based on a deviation between the set pressure and the detected pressure.

Thus, since the vibrator unit 34 is pressed against the pressed region at the set pressure corresponding to each site of a part to be treated inside the body, the ultrasonic output can be precisely detected in the output detection processing and the frequency can be precisely detected.

Therefore, ultrasonic waves having the target frequency can be accurately generated based on the detected frequency. As a result, it can reliably prevent the ultrasonic output from being reduced.

Since the vibrator unit 34 is pressed against the pressed region at the set pressure corresponding to each site of a part to be treated inside the body, the human body is not subjected to the excessive pressure.

The present invention is not limited to each of the above-described embodiments. Numerous modifications and variations of the present invention are possible in light of the spirit of the present invention, and they are not excluded from the scope of the present invention.

## Claims

1. An ultrasonic treatment apparatus comprising:
(a) a treatment vessel for accommodating a medium;
(b) a first ultrasonic generating element disposed at a predetermined location in the treatment vessel; and
(c) a vibrator unit movably disposed in the treatment vessel,
(d) wherein the vibrator unit comprises a housing forming a sealed chamber and having a predetermined location thereof serving as an abutment section to be abutted against a part to be treated, and a second ultrasonic generating element attached to the abutment section in the chamber and having a hollow section formed on a back face thereof.

2. An ultrasonic treatment apparatus comprising:
(a) a treatment vessel for accommodating a medium;
(b) a vibrator unit movably disposed at a predetermined location in the treatment vessel and comprising an ultrasonic generating element;
(c) a driving processing section for driving the ultrasonic generating element to generate ultrasonic waves;
(d) an actuating mechanism for advancing/retracting the vibrator unit;
(e) a pressure detection section disposed in the vibrator unit and for detecting a pressure of the vibrator unit when pressed against a pressed region; and
(f) a pressure adjustment processing section for actuating the actuating mechanism so as to match the pressure detected by the pressure detection section to a set pressure.

3. The ultrasonic treatment apparatus according to claim 2, comprising an output alteration processing section for reading an output of the ultrasonic waves from a driving circuit for driving the ultrasonic generating element, and for driving the ultrasonic generating element so as to match the output of the ultrasonic waves to a target output.

4. The ultrasonic treatment apparatus according to claim 2 or 3, comprising:
(a) a frequency detection section for detecting a frequency of the ultrasonic waves; and
(b) a frequency correction processing section for calculating a correction value of the frequency so as to match the frequency of the ultrasonic waves to a target frequency, sending the correction value to a basic oscillation circuit, and correcting the frequency.

5. The ultrasonic treatment apparatus according to claim 2 wherein the set pressure is preliminarily set corresponding to each site of a part to be treated.
